# EUROPEAN PATENT APPLICATION

(11) **EP 1 281 369 A1**
(43) Date of publication of application: **05.02.2003**
(21) Application number: 02077811.4
(22) Date of filing: 15.07.2002
(51) Int. Cl.: A61B 19/02

(54) **Medical instrument container**

(30) Priority: 02.08.2001 NL 1018695
(71) Applicant: Robouw Csa B.V., 1483 BN De Rijp (NL)
(72) Inventor: van Dijk, Ronald, 1483 ZG De Rijp (NL)
(74) Representative: Ferguson, Alexander

(57) **Abstract**

Holder for tools for surgeries, comprising a tray having a bottom wall and a circumferential wall, both having a permeable or grid-shaped structure, the circumferential wall being built up from several circumferential wall members that can be placed on each other. The circumferential wall members have an upper edge and a lower edge, at least one of which being provided with vertical protrusions to fittingly extend within the lower edge or upper edge, respectively, of a next circumferential wall member.

## Description

The invention relates to a holder for tools for surgeries.

Such holders are known from the supply program of Robouw Medische Techniek B.V. The cassette-like holders have the shape of a tray or box having a bottom wall, a circumferential wall and a lid, which all comprise a stainless steel wire gauze-shaped plate which is surrounded by a round, thickened edge of stainless steel.

On the bottom wall synthetic strips are attached, having apertures for holding tools such that the tools are easily accessible to the surgeon. The position of the tools is determined depending on the moment on which the tool in question is needed. Optionally a separate intermediate bottom can be placed in the cassette, so that there are two layers of tools.

After use in the operating theatre the cassette with contents can be cleaned in a washing machine especially designed to that end and subsequently be sterilised in an autoclave. This usually takes place in so-called central sterilisation departments of hospitals.

The known cassettes have proved themselves in practice. In cases in which many tools are needed, several cassettes are necessary however, that need to be separately fitted out. It then may happen that a cassette is filled for a small part only.

When washing and sterilising the cassettes are placed on each other, as a result of which between two spaces for tools each time two wire gauze plates are present, which may affect the effectiveness of the cleaning.

It is an object of the invention to provide a holder of the type mentioned in the preamble, with which there is much freedom of fitting out and with which a good cleaning is enhanced.

From one aspect the invention to that end provides a holder for tools for surgeries, comprising a tray having a bottom wall and a circumferential wall, both having a permeable or grid-shaped structure, the circumferential wall being built up from several circumferential wall members that can be placed on each other.

One is now able to determine the height of the cassette oneself, adjusted to the number and size/shape of the tools needed for a certain surgery. One can now have the disposal of a large space, in which many tools are present. The distribution of wash water is improved, because there are no double partitions (bottom and lid).

Preferably the circumferential wall members have an upper edge and a lower edge, at least one of which being provided with vertical protrusions to fittingly extend within the lower edge or upper edge, respectively, of a next circumferential wall member. In this way the circumferential wall members are secured with respect to each other in a simple manner and aligned in horizontal direction.

Preferably the vertical protrusions are provided with turned resilient portions for extending over the lower edge or under the upper edge, respectively, of the next circumferential wall member, so that consecutive circumferential wall members are also secured with respect to each other in vertical direction, such that they can also be detached from each other again if so desired.

It is preferred here that the protrusions are provided at the lower edge, so that the upper edge of the top circumferential wall member is free from protrusions.

In order to enhance the firmness, the circumferential wall members are preferably provided with thickened, circumferential lower and upper edges, which are preferably situated within the horizontal profile of the circumferential wall member.

In order to provide more possibilities for fitting out the holder, can be provided with means for support of an insert bottom plate on a circumferential wall member. The insert bottom plate forms an additional surface for tools, but only forms one partition. In a simple embodiment the support means are formed on the protrusions.

Alternatively the support means can be detachably placeable at the inner side of the circumferential wall members, so that there is a large freedom in the placing of an insert bottom plate.

Preferably the holder comprises a fixed bottom tray in which the bottom member is situated. The bottom tray is here provided with handles, so that they are always present, whether or not one or several circumferential wall members have been placed on them.

The versatility is further increased when the bottom tray is provided with means for detachable attachment of a lid, and/or the circumferential wall members are all provided with means for detachable attachment of a lid.

Preferably the holder comprises one or more carriers for tools, which carriers are supported on the circumferential wall members. In this way an insert bottom member could be dispensed with as a result of which the wash water is even better distributed.

It is also possible that the holder is provided with a carrier for tools that is supported on the bottom wall member, which may be advantageous when it regards tools of relatively large dimensions, such as endoscopic tools.

When the said carriers are detachable, the fitting out of the holder can take place more easily.

For the versatility in the assembly of the holder, without creating storage/stocking problems, it is preferred that the circumferential wall members have the same height.

The invention further provides a circumferential wall suitable and intended for a holder according to the invention.

From a further aspect the invention provides a carrier for endoscopic instruments, comprising a frame that can be placed in a tray-shaped holder, for instance a holder according to the invention as described above, the carrier comprising support means for support on the bottom of the tray-shaped holder, as well as rails extending at a distance above the supports, on which rails a number of instrument holders have been arranged and supported in a slidable manner by means of attachment means connected thereto.

Thus it is achieved that the distance one to the other between the instrument holders can easily be adjusted to the instruments needed at that moment. The size of the instruments namely varies: with children shorter instruments are used than is the case with adults. In case of shorter instruments the stability is thus ensured, and space is moreover made available for other instruments.

Preferably the instrument holders are strip-shaped, having a series of accommodation spaces for the instruments. The accommodations spaces may form several partial spaces for accommodating several instruments above each other, so that space is gained. It is preferred here that the accommodation spaces comprise a larger and a smaller partial space, the smaller partial space being situated underneath. The accommodation spaces may then for instance have a substantially tapering shape, both instruments then capable of being held reliably.

It is further preferred that the instrument holders are detachable and capable of being arranged by means of the attachment means on a wire-shaped edge of a tray-shaped holder. Thus one may opt for placing the instrument holders without carrier in a tray-shaped holder, for instance when there is no space (height) for the carrier.

The invention further relates to a holder for endoscopic instruments suitable and intended for a carrier according to the invention.

The invention will be elucidated on the basis of en exemplary embodiment shown in the attached drawings, in which:
Figures 1A-C show a top view, a side view and a front view, respectively, of a bottom tray for a holder according to the invention;
Figures 2A-D show a top view, a side view and a front view, and a detail, respectively, of a circumferential wall member for a holder according to the invention;
Figure 3 shows a holder according to the invention during the assembling with a bottom member and a pair of circumferential wall members according to the figures 1 and 2;
Figure 4 shows an assembled holder according to the invention including tools in it;
Figures 5A-C show a view in perspective, a top view and an end view, respectively, of a carrier according to the invention for endoscopic instruments; and
Figures 6A-C show some possible accommodation spaces for holding endoscopic instruments in the carrier of the figures 5A-C.

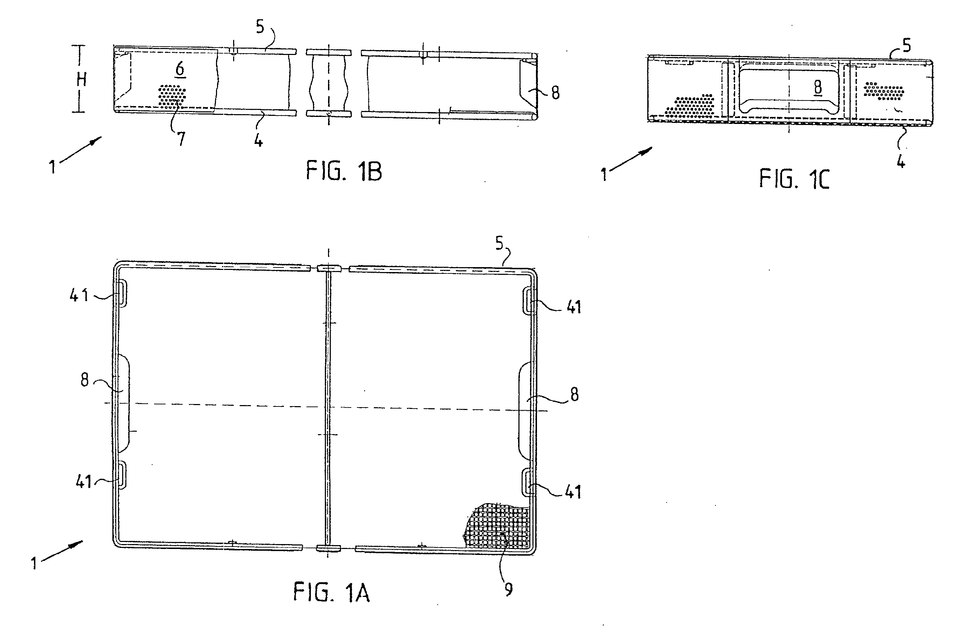

The bottom member 1 of the figures 1A-C comprises a circumferential lower edge 4 and a circumferential upper edge 5, having in between them, circumferential at the outer side thereof, a thin plate 6 provided with holes 7. At the front ends, that means the short sides, recesses 8 have been made, which serve as handles.

On the short sides a number of little brackets 41 have moreover been arranged on the upper edge 5, for an optional lid.

The bottom member 1 is made entirely of stainless steel. The height of the circumferential wall is H.

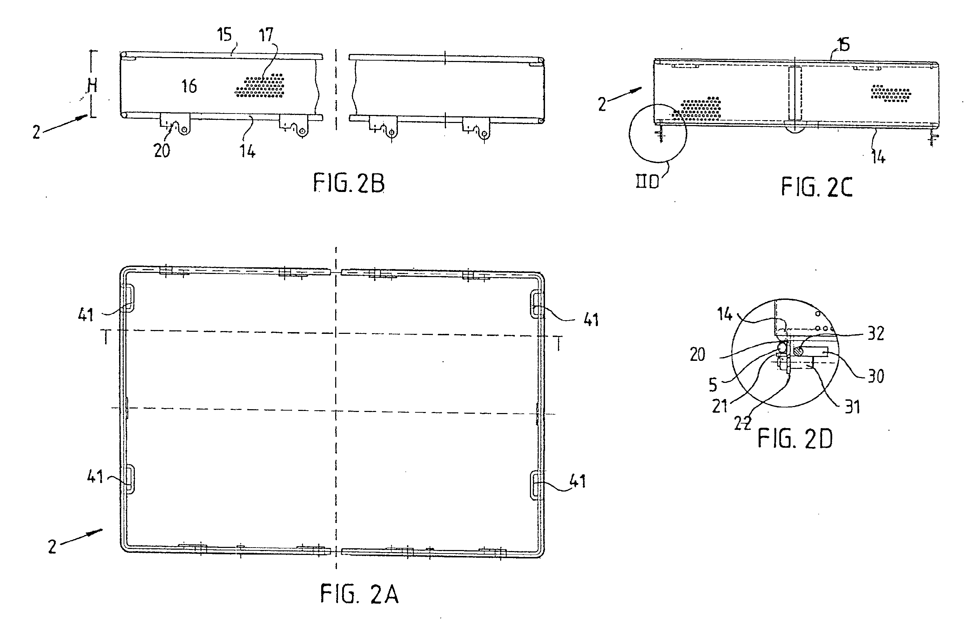

The figures 2A-C substantially correspond to the figures 1A-C. However, it here regards a circumferential wall member 2, which has the same dimensions in top view and in side view, that means it has the same height H as the bottom member 1. The circumferential wall member 2 is also made of stainless steel.

The circumferential wall member 2 thus has a circumferential lower edge 14 and a circumferential upper edge 15, to which at the outer side, a circumferential thin plate 16 with holes 17 has been attached. The circumferential upper edge 15 is also provided with brackets 41 for an optional lid.

At the lower edge 14, as can be seen in figure 2D, lips 20 have been welded to the inner side, which lips extend downward from the lower edge 14 and comprise a lip member 21 that is turned at right angles to the outside and a further downwardly extending lip member 22. The turned lip member 21 serves to engage along the inside and under an upper edge 5 or 15 of a part of the holder according to the invention lying underneath. The lip member 21 may to that end spring to the inside and spring back again.

The lip member 22 is provided with a hole 23, in which if necessary a supporting pin 31 can be attached, on which a separate intermediate bottom 30, provided with circumferential edge 32 and supporting thereon, but not shown, wire gauze material can be laid.

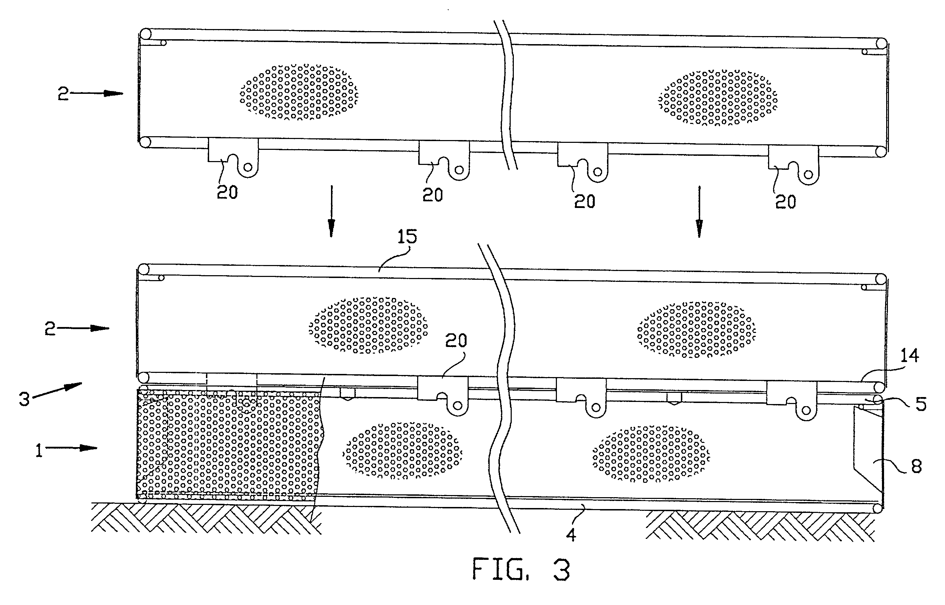

In figure 3 it is shown how a holder 3 according to the invention can be assembled by means of the bottom member 1 and the circumferential wall members 2. The bottom member 1 is placed on a basis, and then a first circumferential wall member 2 is placed thereon, the edge 14 supporting on the edge 5. During placing, the turned lip member 21 is bent inwards in order to subsequently snap under the circumferential wall 5. Thus a unit that can be managed as one unity or holder 3 is obtained. Said unity has a height 2H. If necessary the height can be increased by placing a next circumferential member 2 on the circumferential member 2 as shown in figure 3.

In this way a holder 3 can be obtained with the wanted height, as a multiple of H. The bottom member 1 here always provides the handles 8.

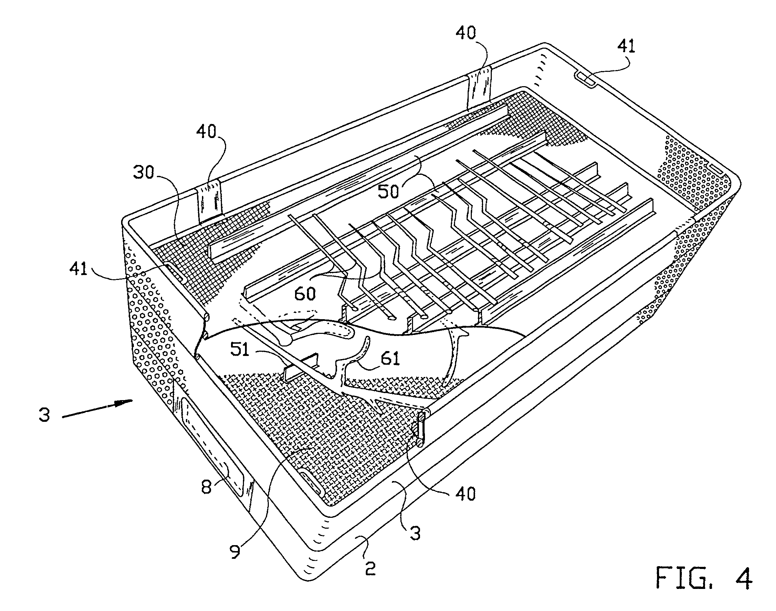

In the manner shown in figure 2D separate bottoms can be placed in between. As can be seen in figure 4, this can also be done in a different way, namely by means of support plates 40 bent in an S-shape, which with their upper part can be hung over the upper edge 15 or 5, and with their lower part, extending inwards, form a support surface for an intermediate bottom 30.

On the bottom surface 9 supports 1? have been attached on which tools 61 can be secured. Above it, on the intermediate bottom 30, supports 50 attached on it are also present, on which further tools 60 can be secured.

The entire holder 3 can be placed in a washing machine, and after that in an autoclave, to clean, disinfect and sterilise the holder plus tools.

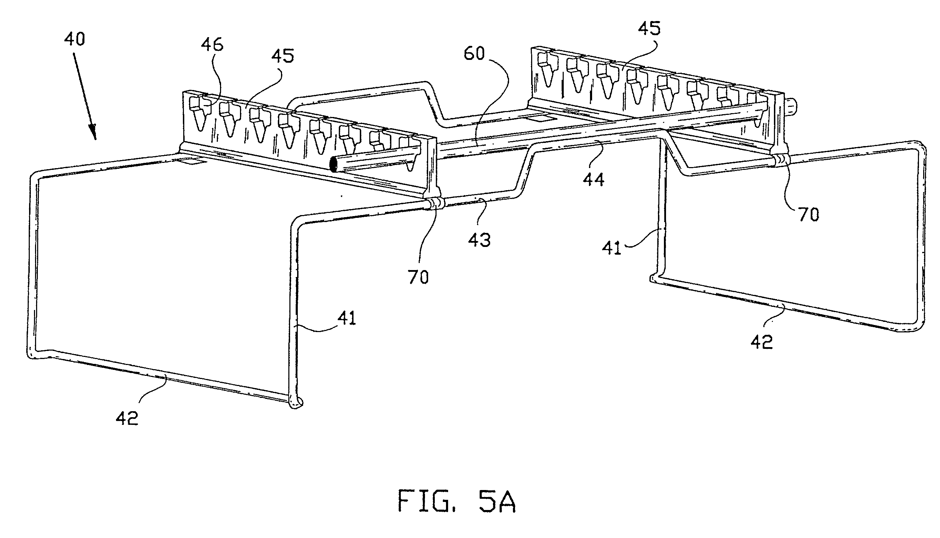

The carrier shown in figures 5A-C is suitable to be fittingly placed in an assembly according to figure 3 and has a stainless steel wire frame 40, having support brackets 41, 42 at both ends which at their upper end change in wire-shaped rails 43, which in their middle have an elevation 44 for engagement by a hand. On the rails 43 instrument holders 45 have been clamped, by means of firm bendable clamping strips 70, and such that the instrument holders 45 can be shifted along the rails 43, to the wanted distance from each other.

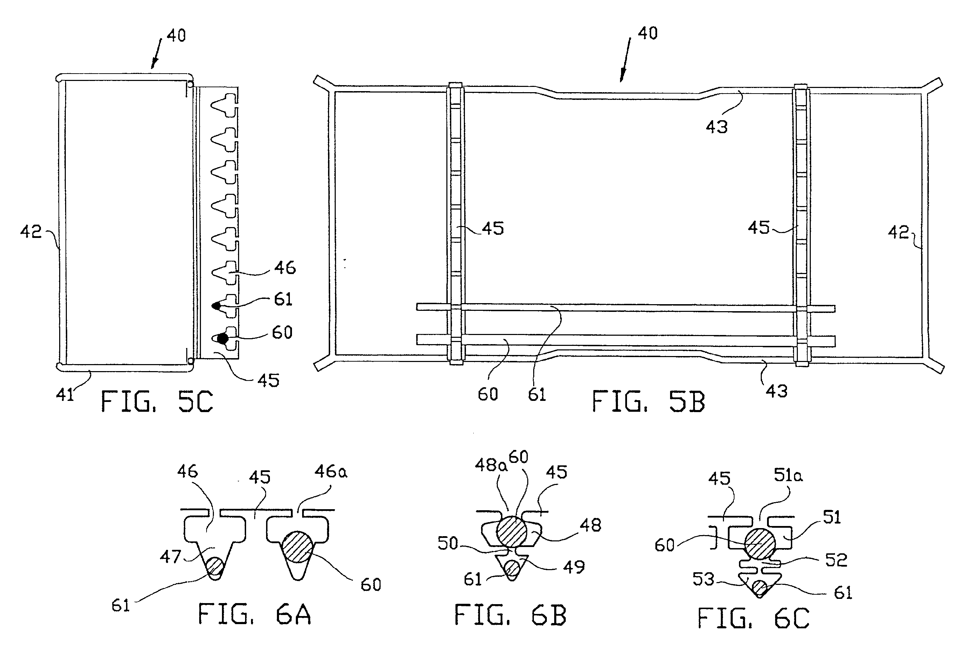

The instrument holders 45 are provided with accommodation spaces 46, which (figure 6A) are accessible from above by means of narrowed passage 46a, the boundaries of which being capable of giving way in an elastic manner during passage, upwards or downwards, of an instrument 60 or 61. The accommodation space 46 downwardly changes into a V-shaped accommodation space 47, in which a thin instrument 61 or a thicker instrument 60 can be held in a reliable manner.

In the figures 6B and 6C it is shown that the accommodations space can also be vertically parted, in which in figure 6B a lower tapering accommodations space 49 is parted by means of a narrowing 50 from a broader accommodation space 48 situated above it, for a thin instrument 61 and a thick instrument 60, respectively. Both accommodation spaces are accessible from the outside first of all via narrowed passage 48a, comparable to passage 46a. In figure 6C a further alternative is shown, the lower accommodation space 53 for a thin instrument 61, and above it accommodation space 51 for a thicker instrument 60, which accommodation space 51 is accessible through passage 51a. Between the accommodation spaces 51 and 53 there is an intermediate space 52, in order to also be able to hold an in-between sized instrument.

The instrument holders 45 are made of silicon material, and may by means of the little clamps 70 also be attached to the lower edges 4 of the bottom member shown in figures 1A-C.

## Claims

1. Holder for tools for surgeries, comprising a tray having a bottom wall and a circumferential wall, both having a permeable or grid-shaped structure, the circumferential wall being built up from several circumferential wall members that can be placed on each other.

2. Holder according to claim 1, the circumferential wall members having an upper edge and a lower edge, at least one of which being provided with vertical protrusions to fittingly extend within the lower edge or upper edge, respectively, of a next circumferential wall member, the vertical protrusions preferably being provided with turned resilient portions for extending over the lower edge or under the upper edge, respectively, of the next circumferential wall member.

3. Holder according to claim 2, the protrusions being provided at the lower edge.

4. Holder according to claims 1, 2 or 3, the circumferential wall members being provided with thickened, circumferential lower and upper edges, which are preferably situated within the horizontal profile of the circumferential wall member.

5. Holder according to any one of the preceding claims, provided with means for support of an insert bottom plate to a circumferential wall member.

6. Holder according to claim 5, the support means being formed at the protrusions.

7. Holder according to claim 5, the support means being detachably placeable at the inner side of the circumferential wall members.

8. Holder according to any one of the preceding claims, the bottom wall member being a part of a fixed bottom tray, the bottom tray preferably being provided with handles and/or with means for detachable attachment of a lid.

9. Holder according to any one of the preceding claims, the circumferential wall members all being provided with means for detachable attachment of a lid.

10. Holder according to any one of the preceding claims, further being provided with one or more carriers for tools which carriers are supported on the circumferential walls.

11. Holder according to any one of the preceding claims, further provided with a carrier for tools, which carrier is supported on the bottom wall member.

12. Holder according to claim 10 or 11, the carrier being detachable.

13. Holder according to any one of the preceding claims, the circumferential wall members having an equal height.

14. Circumferential wall suitable and intended for a holder according to any one of the preceding claims.

15. Carrier for endoscopic instruments, comprising a frame that can be placed in a tray-shaped holder, for instance a holder according to any one of the claims 1-13, the carrier comprising support means for support on the bottom of the tray-shaped holder, as well as rails extending at a distance above the supports, on which rails a number of instrument holders have been arranged and supported in a slidable manner by means of attachment means connected thereto.

16. Carrier according to claim 15, the instrument holders being strip-shaped, having a series of accommodation spaces for the instruments, the accommodations spaces preferably forming several partial spaces for accommodating several instruments above each other, the accommodation spaces preferably comprising a larger and a smaller partial space, the smaller partial space being situated underneath.

17. Carrier according to claim 15 or 16, the instrument holders being detachable and capable of being arranged by means of the attachment means on a wire-shaped edge of a tray-shaped holder.
